(19) Europäisches Patentamt
European Patent Office
Office européen des brevets

(11) Numéro de publication: **0 465 302 B1**

# (12) FASCICULE DE BREVET EUROPEEN

(45) Date de publication de fascicule du brevet: **28.12.94**

(51) Int. Cl.[5]: **C07D 251/34**, C08F 220/36, C08F 220/26, C08G 18/38

(21) Numéro de dépôt: **91401726.4**

(22) Date de dépôt: **26.06.91**

## (54) Précurseurs de polymères à motifs isocyanurates comportant des fonctions hydroxyles, uréthanes, isocyanates ou acryliques et leurs combinaisons, et leurs procédés de fabrication.

(30) Priorité: **02.07.90 FR 9008316**

(43) Date de publication de la demande:
**08.01.92 Bulletin 92/02**

(45) Mention de la délivrance du brevet:
**28.12.94 Bulletin 94/52**

(84) Etats contractants désignés:
**AT BE CH DE DK ES FR GB GR IT LI LU NL SE**

(56) Documents cités:
**US-A- 3 697 584**
**US-A- 4 159 376**

**CHEMICAL ABSTRACTS, vol. 102, no. 2, 14 janvier 1985, page 1, résumé no. 7116a, Columbus, Ohio, US; M. KUCHARSKI et al.: "Addition of epichlorohydrin to tris(2-carboxyethyl) isocyanurate", & ZESZ. NAUK. POLITECH. RZESZOWSKIEJ 1984, 3, 49-54**

**CHEMICAL ABSTRACTS, vol. 80. no. 23, 1974, page 488, résumé no. 133489q, Columbus, Ohio, US; & JP-A-73 26 028 (SHIKOKU KASEI KOGYO CO., LTD) 03-08-1973**

**JOURNAL OF POLYMER SCIENCE, vol. 12, no. 8, 1974, pages 1735-1743, John Wiley & Sons, Inc.; W.J. KAUFFMAN: "Linear polyesters containing isocyanurate rings"**

(73) Titulaire: **ELF ATOCHEM S.A.**
**4 & 8, Cours Michelet**
**La Défense 10**
**F-92800 Puteaux (FR)**

(72) Inventeur: **Grosius, Paul**
**11, rue Jeanne d'Arc**
**F-57540 Petite Rosselle (FR)**
Inventeur: **Vanhoye, Didier**
**9, rue Barabine**
**F-57600 Forbach (FR)**

EP 0 465 302 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention porte sur des précurseurs de polymères à motifs isocyanurates, comportant également des fonctions hydroxyles, uréthanes, isocyanates ou acryliques et des combinaisons de ces fonctions, ainsi que sur leurs procédés de préparation. Ces précurseurs de polymères, appelés dans ce qui suit oligomères, de la présente invention qui comportent des fonctions hydroxyles constituent des composés de départ pour la fabrication d'oligomères de l'invention à fonctions uréthanes, isocyanates ou acryliques et des combinaisons de ces fonctions. Ces oligomères sont intéressants dans la mesure où ils présentent des fonctionnalités variées ; notamment ceux définis au point (2) ci-après sont destinés, entre autres, à être copolymérisés sous rayonnement ultraviolet dans un diluant réactif et en présence d'un initiateur, de manière à former des (co)polymères possédant une bonne dureté et une bonne résistance thermique, utilisables dans le domaine des revêtements, encres et adhésifs photopolymérisables.

Par le brevet américain US-A-4 159 376, on connaît un procédé de préparation d'un isocyanurate à insaturation éthylénique qui consiste à faire réagir un polyisocyanate aromatique avec un monoalcool contenant un groupe vinylidène, choisi notamment parmi le (méth)acrylate d'hydroxypropyle, le (méth)-acrylate d'hydroxyéthyle et leurs mélanges, pour former un uréthane contenant un monoisocyanate, et une seconde étape consistant à faire réagir ledit uréthane avec le tris(hydroxy-2 éthyl)-isocyanurate.

Par le brevet américain US-A-4 485 226, on connaît un procédé de fabrication d'un composé qui réticule sous l'effet d'un rayonnement d'énergie élevée, contenant un groupe isocyanurate, des doubles liaisons oléfiniques et, le cas échéant, des groupes isocyanates bloqués ou libres. Ce procédé comprend la réaction d'un polyisocyanate contenant un groupe isocyanurate avec un composé contenant au moins un groupe réactif avec les groupes isocyanates et au moins une double liaison oléfinique, tel qu'un ester hydroxylé de l'acide acrylique ou méthacrylique avec un dialcool aliphatique ayant une masse moléculaire d'environ 62 - 300, pour former un uréthane, les rapports quantitatifs entre les réactants étant choisis de telle sorte que pour chaque groupe isocyanate libre du polyisocyanate, le mélange réactionnel contienne d'environ 0,9 - 1,5 groupe hydroxyle de l'autre réactif.

Par Journal of Polymer Science, Vol. 12, n° 8, 1974, pages 1735-1743, on connaît des oligomères de formule:

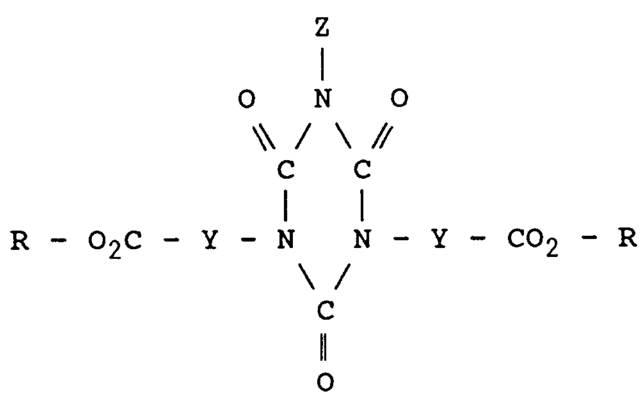

avec Z = $C_6H_5$ ou alkyle ; Y = alkylène ; et R = alkyle ou H. Par US-A-3 697 587, on connaît des composés du type ci-dessus avec Z = $-Y-CO_2-R$.

La présente invention a pour but de proposer de nouveaux oligomères (isocyanato)-uréthano-acryliques à motifs isocyanurates, ainsi que de nouveaux oligomères à motifs isocyanurates comportant des groupes hydroxyle et constituant des réactifs de départ possibles pour la fabrication des nouveaux oligomères précités.

L'invention a donc d'abord pour objet des oligomères renfermant des motifs isocyanurates, représentés par la formule suivante :

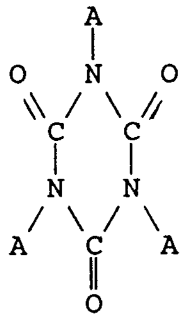

dans laquelle :

(1) au moins un reste A représente un reste

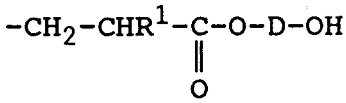 (a)

(où $R^1$ = H ou $CH_3$ et
D = reste alkylène en $C_1$-$C_4$), les autres restes A représentant H ou alkyle en $C_1$-$C_4$, au moins une partie des restes A de formule (a) pouvant être transformée en restes

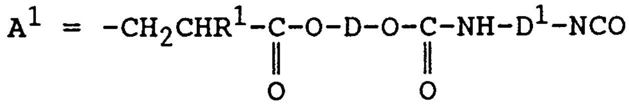

(où $D^1$ est un radical organique divalent exempt de groupes réactifs avec les groupes isocyanate et obtenu par élimination de 2 groupes NCO d'un polyisocyanate saturé ou insaturé, aromatique, aliphatique ou cycloaliphatique, contenant au moins 2 groupes NCO et ne contenant pas de groupes gênant la réaction d'un NCO avec un OH), au moins une partie des restes $A^1$ pouvant être transformée en groupes

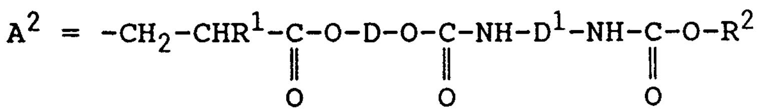

(où $R^2$ est un radical monovalent obtenu par élimination du groupe hydroxyle d'un monoalcool contenant un groupe vinylidène exempt d'autres groupes qui réagissent avec les groupes isocyanate et choisi parmi les produits de réaction d'un acide monocarboxylique contenant un groupe vinylidène avec un dialcool, $R^2$ étant alors représenté par

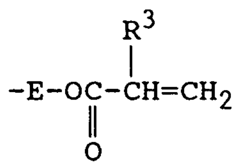

où :

- $R^3$ représente H ou $CH_3$ ; et

- E représente un motif

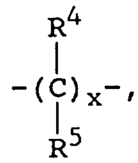

x étant un nombre entier de 1 à 12, et $R^4$ et $R^5$, identiques ou différents, étant choisis parmi l'atome d'hydrogène et des radicaux alkyle ayant 1 à 4 atomes de carbone,

et les hydroxyalkylacrylamides et hydroxyalkylméthacrylamides),

et les mélanges de ces différents composés ;

(2) au moins un reste A représente un groupe de formule:

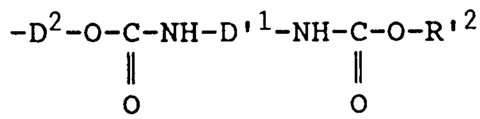 (b)

dans laquelle :

- $D^2$ est un reste alkylène en $C_1$-$C_4$ éventuellement substitué par halogène ; ou un reste $-D^3-(-O-D^3-)_n-$ où $D^3$ est un reste alkylène en $C_1$-$C_4$, éventuellement substitué par halogène, et n est un nombre entier de 1 à 12 ;
- $D'^1$ est analogue à $D^1$ défini ci-dessus ;
- $R'^2$ est analogue à $R^2$ défini ci-dessus

le ou les autres restes A représentant

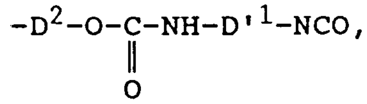

$D^2$ et $D'^1$ étant tels que définis ci-dessus, $D^2$ ne pouvant représenter un reste alkylène en $C_1$-$C_4$ dans le cas où l'oligomère porte les trois groupes (b),

et les mélanges de ces composés.

Un groupe particulier de ces oligomères est celui pour lequel $R^2$ ou $R'^2$ sont issus d'au moins l'un parmi le (méth)acrylate d'hydroxypropyle, le (méth)acrylate d'hydroxyéthyle, et leurs mélanges.

Les oligomères selon la présente invention peuvent se présenter à l'état dilué dans un acrylate multifonctionnel, dans un rapport oligomère/acrylate multifonctionnel d'environ 20 à 80% en poids.

Par acrylate multifonctionnel au sens de la présente invention on entend de manière générale des produits d'estérification de l'acide acrylique ou de l'acide méthacrylique par des polyols, tels que notamment le triméthylolpropaneoxyéthyltriacrylate, le diacrylate du diéthoxyéthyle bis phénol A, le triméthylolpropanetriméthacrylate, l'hexanedioldiacrylate, le tripropylèneglycoldiacrylate, l'éthylglycolacrylate, le pentaérythritoléthoxylé tétraacrylate.

L'invention porte également sur un procédé de préparation des oligomères tels que définis au point (1) ci-dessus, caractérisé par le fait que :

- dans une première étape, on conduit une réaction de Michael entre un composé de formule

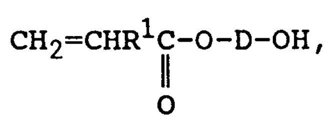

$R^1$ et D étant tels que définis ci-dessus, et l'acide isocyanurique, en catalyse basique, et dans un milieu solvant, pour obtenir un mélange d'oligomères que l'on sépare, le cas échéant, en oligomères individuels ;

- le cas échéant, dans une seconde étape, on conduit une uréthanisation par réaction du ou des oligomères obtenus avec un polyisocyanate OCN-$D^1$-NCO, $D^1$ étant tel que défini ci-dessus ; et
- le cas échéant, dans une troisième étape, on conduit une réaction du produit de l'étape précédente avec un monoalcool $R^2OH$, $R^2$ étant tel que défini ci-dessus ;

puis, le cas échéant, on élimine le solvant.

La réaction de Michael de la première étape est conduite notamment à une température d'environ 60 à 100 ° C, en l'espace d'environ 1 à 4 heures, puis on conduit une cuisson à une température d'environ 110 à 156 ° C, pendant 0,1 à 2 heures, puis on neutralise le catalyseur et on conduit une étape de purification.

Le catalyseur de la réaction de Michael est notamment une amine tertiaire, comme la triéthylamine, un sel d'ammonium quaternaire, une phosphine tertiaire ou une base alcaline, telle que l'hydroxyde de sodium ou l'acétate de sodium.

Le solvant est choisi parmi les solvants aprotiques polaires, comme le diméthylformamide, la N-méthyl pyrrolidine, l'acétate d'éthyle et l'acétonitrile. La purification finale peut consister en une décoloration par charbon actif, une filtration, puis le cas échéant, une évaporation du solvant sous pression réduite.

A la deuxième étape, n'importe quel polyisocyanate tel que défini ci-dessus peut être utilisé, une exigence fondamentale étant que le polyisocyanate ne contienne pas de groupes qui gêneraient la réaction du groupe isocyanate du polyisocyanate avec le groupe hydroxyle du composé ou mélange de composés de la première étape. Des exemples de polyisocyanates qui sont particulièrement utiles dans la présente invention comprennent le 2,4-tolylène diisocyanate, le 2,6-tolylène diisocyanate, le m-phénylène diisocyanate, le p-phénylène diisocyanate, le 1,5-naphtalène diisocyanate, le 1,6-hexaméthylène diisocyanate, le 4,4'-diphényl éther diisocyanate, le 4,4',4''-triphénylméthane triisocyanate, le 2,4,4'-triisocyanato triphényle, le 2,4,4'-triisocyanate diphénylméthane, le 2,4,6-triisocyanato diphényl éther, le 2,2',4-triisocyanate diphényl éther, le 2,2',4-triisocyanate diphényl sulfure, le 2,4,4'-triisocyanato diphényl sulfure, le 2,3',4-triisocyanato-4'-méthyl diphényl éther, le 2,3',4-triisocyanato-4'-méthoxy diphényl éther, le 2,4,4'-triisocyanato-3'-chlorodiphényléther, le 4,4',6-diphényl triisocyanate, le 1,10-décaméthylène diisocyanate, le cumène-2,4-diisocyanate, le 4-méthoxy-1,3-phénylène diisocyanate, le 4-chloro-1,3-phénylène diisocyanate, le 4-bromo-1,3-phénylène diisocyanate, le 4-éthoxy-1,3-phénylène diisocyanate, le 2,4'-diisocyanato diphényl éther, le 5,6-diméthyl-1,3-phénylène diisocyanate, le benzidine diisocyanate, le 9,10-anthracéine diisocyanate, le 4,6-diméthyl-1,3-phénylène diisocyanate, le 4,4'-diisocyanatodibenzyl, le 3,3'-diméthyl-4,4'-diisocyanato diphényl méthane, le 2,6-diméthyl-4,4'-diisocyanato diphényle, le 2,4-diisocyanato stilbène, le 3,3'-diméthyl-4,4'-diisocyanato diphényle, le 3,3'-diméthoxy-4,4'-diisocyanatodiphényle, le 1,4-anthracène diisocyanate, le 2,6-fluorène diisocyanate, le 1,8-naphtalène diisocyanate, le 2,6-diisocyanato benzofuranne, le 2,4,6-tolylène triisocyanate, le 2,4,4'-triisocyanato diphényl éther, le diphénylméthane polyisocyanate disponible sous la marque de fabrique Mondur MR ayant une fonctionnalité de 2,6; et le 1,3-xylène-4,6-diisocyanate. Une classe préférée de polyisocyanates est constituée par les polyisocyanates aromatiques. Des polyisocyanates préférés sont le 2,4-tolylène diisocyanate, le 2,6-tolylène diisocyanate, le tolylène diisocyanate commercial (un mélange de 80% de l'isomère-2,4 et de 20% de l'isomère-2,6), le méthylène bis (p-phénylisocyanate), le 1,3-cyclopentylène diisocyanate, le 2,4,6-toluène triisocyanate, le p-xylylène diisocyanate, le tétraméthylène diisocyanate, l'hexaméthylène diisocyanate, le 2,2,4-triméthylhexaméthylène diisocyanate, le 2,4,4'-triméthylhexaméthylènediisocyanate, le 3-isocyanatométhyl-3,5,5-triméthyl cyclohexyl diisocyanate, le bis(2-isocyanatoéthyl carbonate), les prépolymères à terminaison isocyanate de diols et triols, et les prépolymères à terminaison isocyanate de polyesters.

Les conditions de cette deuxième étape d'uréthanation sont les conditions classiques dans la technique pour la réaction d'un alcool avec un isocyanate pour former un uréthane, c'est-à-dire une température comprise entre 20 ° C et 120 ° C environ, avec utilisation possible de catalyseurs tels que l'octoate ou le dibutyldilaurate d'étain, et d'un solvant aprotique polaire ne possédant pas de fonction susceptible de réagir avec la fonction isocyanate.

Les monoalcools $R^2OH$ qui sont utiles dans la troisième étape du procédé précité comprennent n'importe quel monoalcool contenant un groupe vinylidène ($CH_2 = C<$) et qui est exempt de groupes, autres que le groupe hydroxyle, qui sont réactifs avec les groupes isocyanate. Une classe préférée de monoalcools est préparée en faisant réagir un acide monocarboxylique contenant un groupe vinylidène avec un dialcool. Des exemples illustratifs de tels acides comprennent l'acide acrylique, l'acide méthacrylique, l'acide éthacrylique, l'acide 9,12-octadécadiénoïque, l'acide 9,12,15-octadécatriénoïque, l'acide 9,11,13-octadécatriénoïque, et l'acide 4-céto-9,11,13-octadécatriénoïque. Des exemples illustratifs de dialcools comprennent l'éthylèneglycol, le propylèneglycol, le diéthylèneglycol, le dipropylèneglycol, le 1,3-butylèneglycol, le 1,4-butylèneglycol, le 2,3-butylèneglycol, le pentaméthylèneglycol, l'hexaméthylèneglycol, le néopentylglycol, le dibromonéopentylglycol, le diméthylhexènediol, le diméthylhexanediol, le 2-butène-1,4-diol, le 2-butane-1,4-diol, le 2,3-dibromo-2-butène-1,4-diol, le 2,2,3,3-tétrachloro-1,4-butanediol, le 2,2,4-triméthyl-1,6-hexanediol, le 2,5-diméthyl-2,5-hexanediol, le 2,2,4,4-tétraméthyl-1,3-cyclobutanediol, le 1,4-cyclohexanediol, le 1,4-cyclohexanediméthanol, le bisphénol A hydrogéné, et les éthers d'oxyde d'éthylène et/ou oxyde de propylène des diols mentionnés ci-dessus. Un groupe préféré de monoalcools qui sont utiles dans le procédé de cette invention comprend le méthacrylate d'hydroxypropyle, le méthacrylate d'hydroxyéthyle, l'acrylate d'hydroxyéthyle et l'acrylate d'hydroxypropyle, et leurs mélanges. Par ailleurs, comme déjà indiqué ci-dessus, le monoalcool $R^2OH$ peut être constitué par un hydroxyalkylacrylamide ou un hydroxyalkyméthacrylamide.

Les conditions réactionnelles de cette troisième étape sont, d'une manière générale, identiques à celles de la deuxième étape. Etant donné la nature insaturée du monoalcool $R^2OH$, il est cependant préférable d'opérer dans des conditions empêchant toute polymérisation, par exemple en procédant à un bullage d'air sec et/ou en opérant en présence d'une quantité efficace d'au moins un inhibiteur de polymérisation. Comme exemples d'inhibiteurs de polymérisation utilisables, on peut citer notamment la phénothiazine, l'éther méthylique de l'hydroquinone, la N,N-diéthylhydroxylamine, le nitrobenzène, le di-tertio-butylcatéchol, l'hydroquinone, le p-anilinophénol, le phosphite de di-(2-éthylhexyl)-octylphényle, le 2,5-ditertiobutyl-4-hydroxytoluène,le bleu de méthylène et leur mélanges en toutes proportions. Une quantité efficace d'inhibiteur de polymérisation est généralement comprise entre 0,05% et 0,5% en poids des composés polymérisables.

L'invention porte également sur un procédé de préparation des oligomères tels que définis au point (2) ci-dessus, dans lesquels au moins un reste A représente un groupe de formule :

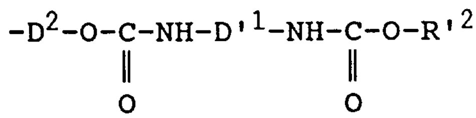 (b)

dans laquelle :

- $D^2$ est un reste alkylène en $C_1$-$C_4$ éventuellement substitué par halogène ; ou un reste $-D^3-(-O-D^3-)_n-$ où $D^3$ est un reste alkylène en $C_1$-$C_4$, éventuellement substitué par halogène, et n est un nombre entier de 1 à 12 ;
- $D'^1$ est analogue à $D^1$ défini ci-dessus ;
- $R'^2$ est analogue à $R^2$ défini ci-dessus ;

le ou les autres restes A représentant

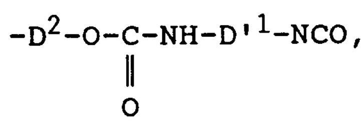

$D^2$ et $D'^1$ étant tels que définis dans le présent paragraphe, caractérisé par le fait que :

- dans une première étape, on fait réagir un composé de formule :

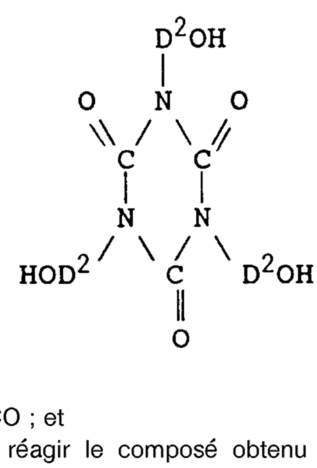

  avec un polyisocyanate OCN-$D'^1$-NCO ; et
- dans une deuxième étape, on fait réagir le composé obtenu dans lequel tous les $D^2OH$ ont été remplacés par

$$D^{2}O\underset{\substack{\| \\ O}}{C}-NH-D'^{1}-NCO$$

avec un composé de formule $R'^2OH$, dans un rapport molaire de 1 à 3 de $R'^2OH$ au composé précédent.

Les polyisocyanates et les composés hydroxylés $R'^2OH$ sont choisis parmi ceux indiqués ci-dessus en référence au premier procédé. Les conditions réactionnelles sont, de la même façon, celles d'une uréthanation classique.

Au moins une partie du solvant utilisé est avantageusement un acrylate multifonctionnel tel que défini précédemment.

Enfin la présente invention porte sur tout polymère ou copolymère renfermant dans sa chaîne au moins un motif dérivé d'un oligomère à motif isocyanurate tel que décrit précédemment. Un tel copolymère peut en outre comprendre des motifs dérivés d'au moins un comonomère copolymérisable avec ledit oligomère, tel que notamment :

- un acrylate multifonctionnel tel que défini ci-dessus,
- un acrylate ou méthacrylate d'alkyle dont le groupe alkyle, linéaire ou ramifié, possède de 1 à 20 atomes de carbone, et
- un acrylate ou méthacrylate d'aryle tel que le méthacrylate de benzyle,
- un hydrocarbure vinylaromatique tel que le styrène, le vinyltoluène, l'alphaméthylstyrène, le méthyl-4 styrène, le méthyl-3 styrène, le méthoxy-4 styrène et le vinyl-1-naphtalène.

Dans les exemples suivants, destinés à illustrer l'invention, les pourcentages sont donnés en poids sauf indication contraire, et l'on a utilisé les abréviations suivantes :

AC : acide isocyanurique
Triton B : hydroxyde de benzyltriméthylammonium
TEBAC : chlorure de triéthylbenzylammonium
HEA : acrylate d'hydroxyéthyle
HEMA : méthacrylate d'hydroxyéthyle
HPA : acrylate d'hydroxypropyle
HPMA : méthacrylate d'hydroxypropyle
EMHQ : éther méthylique de l'hydroquinone
PTZ : phénothiazine
THPIC : trishydroxypropylisocyanurate
TDI : toluènediisocyanate
IPDI : isophoronediisocyanate
HMDI : hexaméthylènediisocyanate
HDDA : hexanedioldiacrylate
TMPEOTA : triméthylolpropaneoxyéthylènetriacrylate
TPGDA : tripropylèneglycoldiacrylate
AcOEt : acétate d'éthyle
$Bu_2SnLau_2$ : dilaurate de dibutylétain
ACN : acrylonitrile
NEMA : hydroxyalkylméthacrylamide
NEtz = triéthylamine

Exemples 1 à 12 :

Préparation d'un mélange d'oligomères hydroxylés possédant un noyau isocyanurate par addition de Michael de l'acide isocyanurique sur l'acrylate d'hydroxy-2 éthyle.

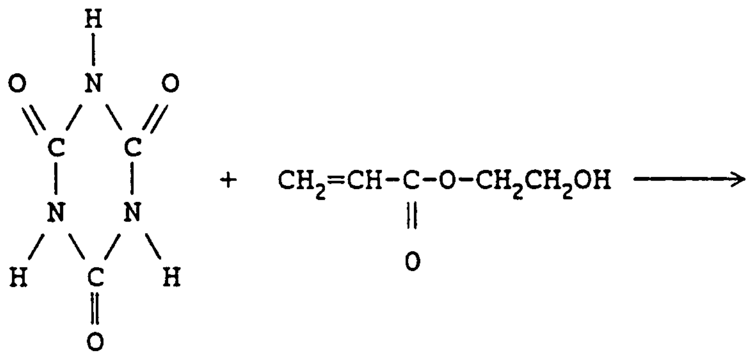

mélange de trois composés de formule générale :

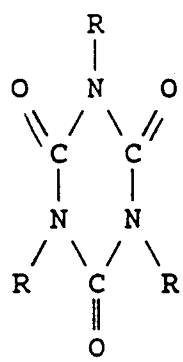

(1) l'un des R représente

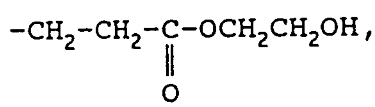

et les deux autres, H (dérivé mono) ;
(2) deux R représentent chacun

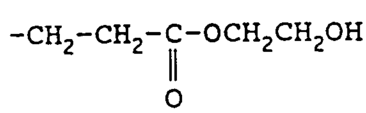

et l'autre, H (dérivé di) ; et
(3) les trois R représentent

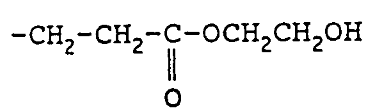

(dérivé tri).

Mode opératoire général

Dans un réacteur de 1 l, muni d'une agitation mécanique centrale et d'un condenseur à reflux, on introduit 375 ml de diméthylformamide, 64,5 g (0,5 mole) d'acide isocyanurique, une quantité donnée de catalyseur (Triton B ou TEBAC), 2000 ppm d'EMHQ par rapport à l'acide isocyanurique et 1000 ppm de PTZ par rapport à l'acide isocyanurique.

On porte l'ensemble à la température $T_1$, sous bullage d'air. On introduit alors une quantité donnée d'acrylate d'hydroxyéthyle à l'aide d'une pompe doseuse GILSON en un temps $t_1$.

L'addition terminée, on augmente la température du milieu jusqu'à $T_2$ et on laisse agiter pendant un temps $t_2$. Puis, on refroidit le contenu du réacteur et on vidange celui-ci.

On neutralise alors le catalyseur par une solution d'HCl 1N, puis on traite le mélange réactionnel coloré en brun orangé sur du charbon actif (0,5% par rapport à la masse réactionnelle ; 80 ° C ; 1 heure), puis on le filtre sur Celite. Enfin, on concentre le filtrat à l'évaporateur rotatif (60 ° C ; 400 Pa - 3mmHg) pour éliminer le diméthylformamide. On obtient un produit d'addition constitué par un mélange de dérivés mono, di et tri.

Les différentes conditions opératoires et les résultats obtenus figurent au Tableau 1 ci-après. Les mélanges obtenus sont utilisables, sans autre purification, pour la synthèse d'oligomères uréthano par réaction avec un diisocyanate (rapport NCO/OH de 2), puis d'oligomères uréthano-acryliques par réaction de ces derniers avec un (méth)acrylate d'hydroxyalkyle.

Tableau 1

| Ex | $t_1$ (h) | Cataly-seur | Rapport molaire cataly-seur/AC | Rapport molaire HEA/AC | $T_1$ (°C) | $T_2$ (°C) | $t_2$ (h) | Mono % | Di % | Tri % | HEA % | Masse g | R Mono | R Di | R Tri | ΣR % |
|---|---|---|---|---|---|---|---|---|---|---|---|---|---|---|---|---|
| 1 | 1 | TriB | 1,5 | 3,05 | 80 | | | 7,9 | 17,6 | 36,6 | >4,6 | 224,5 | 14,5 | 21,9 | 34,5 | 70,9 |
| 2 | 4 | " | " | 2,8 | 50 | | | 9,1 | 18,1 | 32,5 | >4,6 | 232 | 17,2 | 23,3 | 31,6 | 72,1 |
| 3 | 1 | TEBAC | " | 2,8 | 80 | | | 11,9 | 13,3 | 14,6 | >4,6 | 210 | 20,4 | 15,5 | 12,9 | 48,8 |
| 4 | 4 | " | " | 3,05 | 50 | | | 7,9 | 8,8 | 13,3 | 4,9 | 216 | 13,9 | 10,5 | 12,0 | 36,4 |
| 5* | 1 | TriB | 3,1 | 3,05 | 50 | | | 7,7 | 17,7 | 35,1 | >4,6 | 412,8 | 13,0 | 20,2 | 30,4 | 63,6 |
| 6 | 4 | " | " | 2,8 | 80 | 156 | 1 | 6,2 | 18,3 | 44,5 | 3,9 | 219 | 11,0 | 22,2 | 40,9 | 74,2 |
| 7 | 1 | TEBAC | " | " | 50 | | | 10,2 | 11,0 | 16,9 | 7,2 | 210 | 17,5 | 12,8 | 15,3 | 45,6 |
| 8 | 4 | " | " | " | 80 | | | 13,1 | 12,9 | 14,2 | 5,4 | 203 | 21,7 | 14,5 | 12,1 | 48,3 |
| 9 | 4 | TriB | " | " | " | 120 | 1 | 15,4 | 24,1 | 40,3 | 14,4 | 209,5 | 27,0 | 28,0 | 35,4 | 90,4 |
| 10 | 4 | " | " | " | " | " | 0,5 | 17,2 | 24,3 | 41,8 | 16,1 | 208,2 | 29,3 | 28,0 | 36,5 | 93,8 |
| 11 | 4 | " | " | " | " | " | 1 | 14,9 | 22,7 | 48,5 | 13,0 | 202,5 | 24,6 | 25,5 | 41,1 | 91,2 |
| 12 | 4 | " | " | " | " | " | 1 | 25,8 | 25,0 | 29,5 | 1,2 | 165 | 34,8 | 22,9 | 20,4 | 78,1 |

* AC = 0,5 mole

$$R_{\text{mono, di ou tri}} = \frac{\text{Nombre de moles de mono- di ou tri formé}}{\text{Nombre de moles AC de départ}} \times 100 \qquad \Sigma R = R_{mono} + R_{di} + R_{tri}$$

Exemples 13 à 21

(1) Préparation d'un oligomère isocyanato uréthano

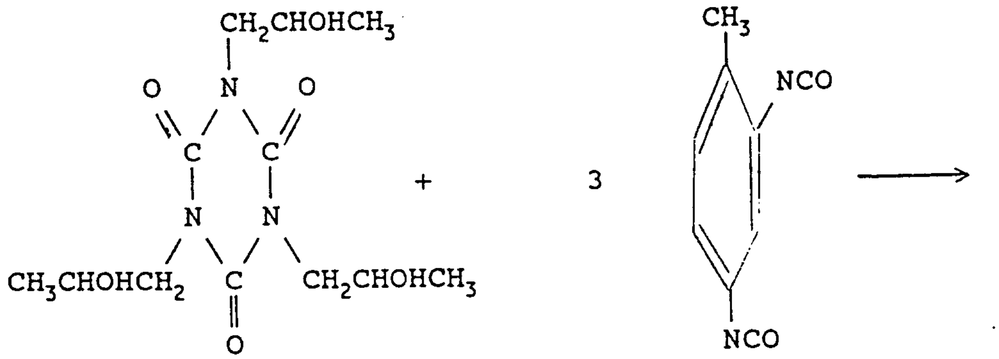

THPIC

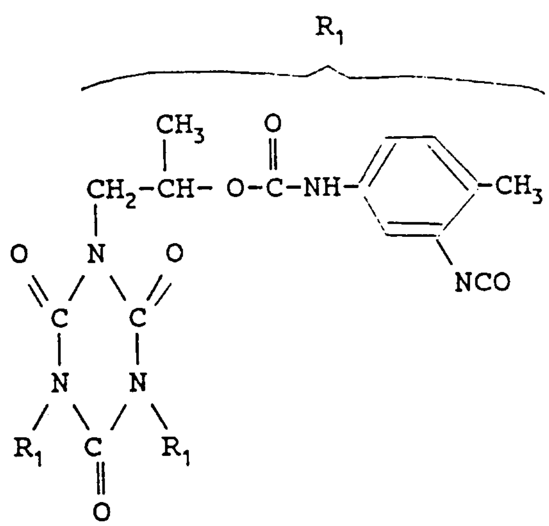

Dans un réacteur de 250 ml, muni d'une agitation mécanique centrale et d'un réfrigérant ascèndant, on introduit 30,3 g (0,1 mole) de poudre de THPIC, 82,5 g d'acétate d'éthyle anhydre et 52,2 g (0,3 mole) de TDI. On place ensuite le milieu réactionnel sous balayage d'azote sec, puis on porte la température à 60 ° C pendant 3 heures, temps au bout duquel le taux de transformation des NCO est voisin de 50%. Le milieu réactionnel est alors refroidi à 30 ° C.

(2) Acrylation de l'oligomère obtenu en (1)

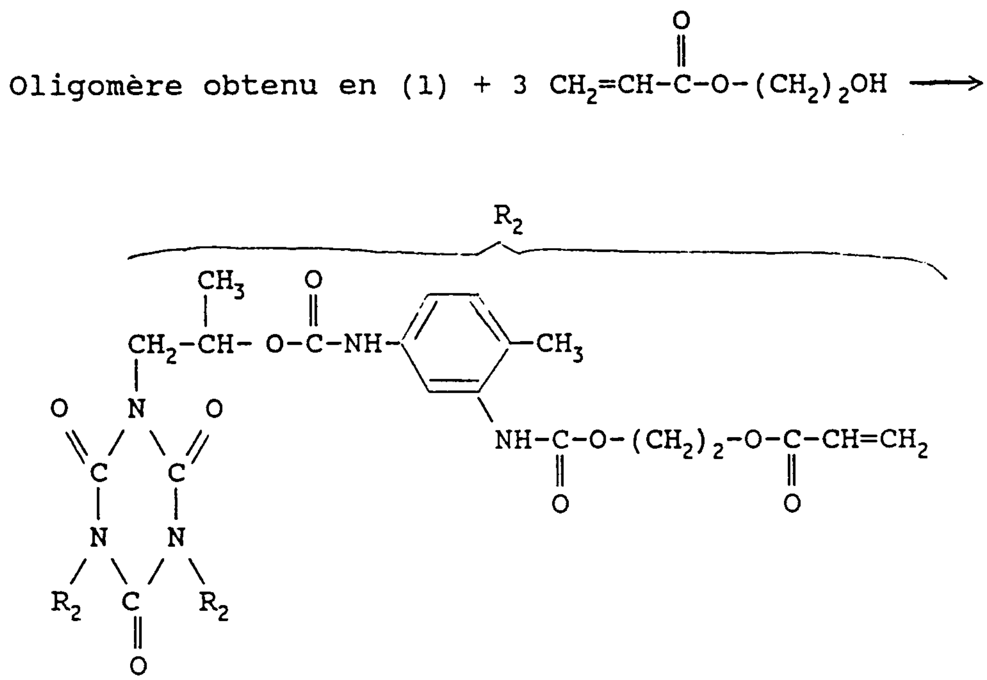


Quand la température de 30 °C du milieu réactionnel de l'étape 1 est atteinte, on introduit 33,06 g (0,3 mole) d'acrylate d'hydroxy-2 éthyle, 0,038 g de $Bu_2SnLau_2$, le cas échéant 0,006 g de $NEt_3$, et 0,05 g d'EMHQ (1500 ppm/HEA). On porte alors le mélange à T °C, sous bullage d'air sec, pendant t heures, puis on le refroidit jusqu'à la température ambiante. On vidange ensuite le mélange, on le filtre sur papier en cas de présence d'insolubles, puis on le stocke à l'abri de la lumière et à une température inférieure à 20 °C.

Les résultats sont rapportés dans le Tableau 2.

Tableau 2

| Ex | Solvant | ES(%) * | Catalyseur | Rapport molaire catalyseur/oligomère obtenu en (1) $10^{-4}$ | T(°C) | t(h) | Mole NCO pour 100 g $10^{-3}$ | TT NCO (%) ** | % HEA | TT HEA (%) ** | η (Pa.s) |
|---|---|---|---|---|---|---|---|---|---|---|---|
| 13 | HDDA | 25 | $Bu_2SnLau_2$ $NEt_3$ | 6 | 80 | 3 | 8,1 | 95,0 | 4,9 | 50 | 0,36 |
| 14 | AcOEt | 50 | " | 3 | 100 | 6 | 1,1 | 99,6 | 9,4 | 46,9 | 17,2 |
| 15 | AcOEt | 25 | $Bu_2SnLau_2$ | 6 | 100 | 6 | 1,0 | 99,4 | 2,4 | 73,9 | 0,15 |
| 16 | HDDA | 25 | " | 3 | 80 | 6 | 7,9 | 99,5 | 5,3 | 45,8 | 0,30 |
| 17 | HDDA | 50 | " | 3 | 100 | 3 | 1,3 | 99,6 | 3,4 | 79,7 | 63 *** |
| 18 | HDDA | 50 | $Bu_2SnLau_2$ $NEt_3$ | 6 | 80 | 3 | 4,8 | 98,4 | 5,4 | 67,6 | 2,5 |

* ES = extrait sec

** TT = taux de transformation

*** viscosité limite pouvant être mesurée par l'appareil

Exemples 23 à 37

(a) Préparation d'un oligomère d'addition THPIC + diisocyanate

Dans un réacteur de 250 $cm^3$, on introduit, à 20 ° C, 0,3 mole de diisocyanate, 0,1 mole de THPIC, une quantité appropriée de solvant pour avoir un extrait sec théorique de 50%, 3 x $10^{-4}$ mole de $Bu_2SnLau_2$ par mole de THPIC dans le cas de l'IPDI et du HMDI.

Le contenu du réacteur est alors porté à 60 ° C, sous agitation pendant 2 heures, conduisant à un pourcentage de fonctions isocyanates résiduelles voisin de 50%.

Les résultats sont rapportés dans le Tableau 4.

Tableau 4

| Ex | Solvant | Diisocyanate | Catalyseur | $NCO_R/NCO_{in}$ | | | Masse théorique oligomère (g) | $\eta$ (Pa.s) |
|---|---|---|---|---|---|---|---|---|
| | | | | To | 1h | 2h | | |
| 23a | AcOEt | TDI | - | 96,2 | 65,3 | 56,9 | 825 | 0,019 |
| 24a | AcN | TDI | - | | 54,6 | 54,4 | 825 | - |
| 25a | AcOEt | HMDI | - | | 91,5 | 91,0 | 807 | - |
| 26a | AcOEt | HMDI | $Bu_2SnLau_2$ | | 45,7 | - | 807 | 0,033 |
| 27a | AcOEt | IPDI | - | | 100 | 100 | 969 | - |
| 28a | AcOEt | IPDI | $Bu_2SnLau_2$ | | 58,8 | 49,6 | 969 | 0,036 |
| *T = 80 ° C | | | | | | | | |

(b) Réaction de l'oligomère obtenu en (a) avec un dérivé acrylique hydroxylé

On introduit, à 20 ° C, dans le mélange précédent, 0,1 mole à 0,3 mole de dérivé acrylique hydroxylé, et 1000 ppm d'EMHQ par rapport au dérivé acrylique, puis le contenu du réacteur est porté à 60 ° C pendant 2 heures (ou plus) pour conduire au taux de transformation désiré. Les résultats sont rapportés dans le Tableau 5.

Tableau 5

| Ex | Oligomère obtenu en (a) | Monomère acrylique | Rapport molaire dérivé acrylique/oligomère obtenu en (a) | $NCO_R/NCO_{in}$ * | | | ES théorique (%) | Masse théorique oligomère (g) | η (Pa.s) |
|---|---|---|---|---|---|---|---|---|---|
| | | | | 1h | 2h | Final | | | |
| 23b | Ex 23a | HEMA | 2 | 28,9 | 21,3 | 16,1 | 56,8 | 1085 | 0,170 |
| 30b | Ex 23a | HEA | 2 | 18,2 | 14,6 | 14,6 | 56,5 | 1071 | 0,180 |
| 31b | Ex 23a | HPMA | 2 | - | 29,7 | 20,0 | 57,1 | 1099 | 0,44 |
| 32b | Ex 23a | HPA | 2 | 30,5 | 24,7 | 21,4 | 56,8 | 1083 | 0,09 |
| 33b | Ex 23a | NEMA | 2 | 29,2 | 26,4 | 19,1 | 56,8 | 1083 | 0,44 |
| 34b | Ex 23a | HEMA | 1 | 33,5 | 31,6 | 31,6 | 53,6 | 955 | 0,050 |
| 36b | Ex 26a | HEMA | 2 | 13,3 | 12,8 | 12,8 | 56,9 | 1067 | 0,240 |
| 37b | Ex 28a | HEMA | 2 | 33,4 | - | 28,6 | 55,9 | 1229 | 0,007 |

* $NCO_R/NCO_{in}$ : NCO résultant/NCO initial

- : non déterminé

Exemples 38 à 44

Mode opératoire général

L'oligomère (isocyanato)-uréthano-acrylique en solution dans l'acétate d'éthyle est mélangé à un diluant réactif. Le solvant est ensuite soumis à un strippage à l'évaporateur rotatif (50 °C ; 5,3 x $10^3$ Pa), pour donner une composition (isocyanato)-uréthano-acrylique contenant 30% en masse d'oligomère de départ.

Les résultats sont rapportés dans le Tableau 6

Tableau 6

| Ex | Oligomère | Diluant Réactif | Viscosité à 25 °C (Pa.s) | Nbre mole NCO pour 100 g solution |
|---|---|---|---|---|
| 38 | THPIC + TDI + 2HEA de l'ex. | TMPEOTA | 2,51 | 8,5 x $10^{-3}$ |
| 39 | " | TPGDA | 0,61 | 2,2 x $10^{-2}$ |
| 40 | " | HDDA | 0,13 | 6 x $10^{-2}$ |
| 41 | THPIC + IPDI + 2HEA de l'ex 37b | TMPEOTA | 1,33 | 0,66 |
| 42 | THPIC + TDI + 2HPMA de l'ex 31b | " | 2,72 | 5,5 x $10^{-2}$ |
| 43 | THPIC + TDI + 2HPA de l'ex 32b | " | 1,74 | 5,6 x $10^{-2}$ |
| 44 | THPIC + TDI + 2HEMA de l'ex 29b | " | 2,35 | 2,5 x $10^{-2}$ |

## Revendications

**Revendications pour les Etats contractants suivants : AT, BE, CH, DE, DK, FR, GB, GR, IT, LI, LU, NL, SE**

**1.** Oligomères renfermant des motifs isocyanurates, représentés par la formule suivante :

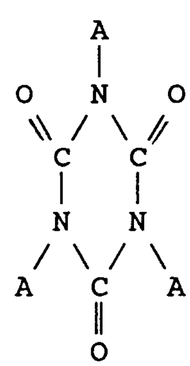

dans laquelle :

(1) au moins un reste A représente un reste

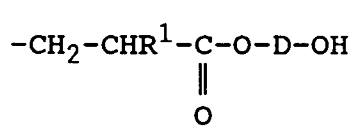 (a)

(où $R^1$ = H ou $CH_3$ et

D = reste alkylène en $C_1$-$C_4$), les autres restes A représentant H ou alkyle en $C_1$-$C_4$, au moins une partie des restes A de formule (a) pouvant être transformée en restes

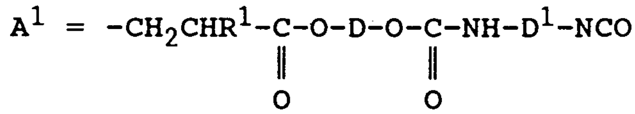

(où $D^1$ est un radical organique divalent exempt de groupes réactifs avec les groupes isocyanate et obtenu par élimination de 2 groupes NCO d'un polyisocyanate saturé ou insaturé. aromatique, aliphatique ou cycloaliphatique, contenant au moins 2 groupes NCO et ne contenant pas de groupe gênant la réaction d'un NCO avec un OH), au moins une partie des restes $A^1$ pouvant être transformée en groupes

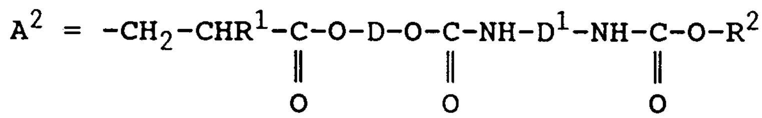

(où $R^2$ est un radical monovalent obtenu par élimination du groupe hydroxyle d'un monoalcool contenant un groupe vinylidène exempt d'autres groupes qui réagissent avec les groupes isocyanate et choisi parmi les produits de réaction d'un acide monocarboxylique contenant un groupe vinylidène avec un dialcool, $R^2$ étant alors représenté par

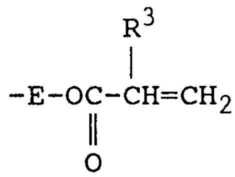

où :

- $R^3$ représente H ou $CH_3$ ; et
- E représente un motif

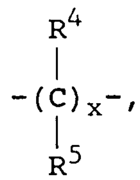

x étant un nombre entier de 1 à 12, et $R^4$ et $R^5$, identiques ou différents, étant choisis parmi l'atome d'hydrogène et des radicaux alkyle ayant 1 à 4 atomes de carbone,

et les hydroxyalkylacrylamides et hydroxyalkylméthacrylamides),
et les mélanges de ces différents composés ;
(2) au moins un reste A représente un groupe de formule:

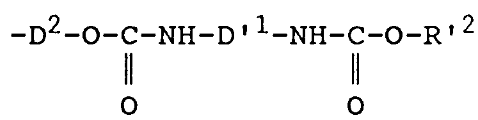 (b)

dans laquelle :

- $D^2$ est un reste alkylène en $C_1$-$C_4$ éventuellement substitué par halogène ; ou un reste $-D^3-(-O-D^3-)_n-$ où $D^3$ est un reste alkylène en $C_1$-$C_4$, éventuellement substitué par halogène, et n est un nombre entier de 1 à 12 ;
- $D'^1$ est analogue à $D^1$ défini ci-dessus ;

- $R'^2$ est analogue à $R^2$ défini ci-dessus ;

le ou les autres restes A représentant

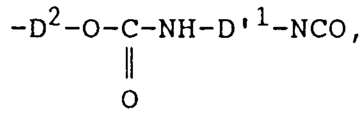

$D^2$ et $D'^1$ étant tels que définis ci-dessus, $D^2$ ne pouvant représenter un reste alkylène en $C_1$-$C_4$ dans le cas où l'oligomère porte les trois groupes (b),
et les mélanges de ces composés.

**2.** Oligomères selon la revendication 1, caractérisés par le fait que $D_1$ est un radical obtenu par élimination de 2 groupes NCO d'un polyisocyanate aromatique.

**3.** Oligomères selon l'une des revendications 1 et 2, caractérisés par le fait que $R^2$ ou $R'^2$ sont issus d'au moins l'un parmi le (méth)acrylate d'hydroxypropyle, le (méth)acrylate d'hydroxyéthyle, et leurs mélanges.

**4.** Oligomères selon l'une des revendications 1 à 3, caractérisés par le fait qu'ils se présentent à l'état dilué dans un acrylate multifonctionnel, dans un rapport oligomère/acrylate multifonctionnel de 20 à 80% en poids.

**5.** Procédé de préparation des oligomères tels que définis au point (1) de la revendication 1, caractérisé par le fait que :

- dans une première étape, on conduit une réaction de Michael entre un composé de formule

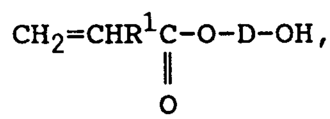

  $R^1$ et D étant tels que définis à la revendication 1, et l'acide isocyanurique, en catalyse basique, et dans un milieu solvant, pour obtenir un mélange d'oligomères que l'on sépare, le cas échéant, en oligomères individuels ;
- le cas échéant, dans une seconde étape, on conduit une uréthanisation par réaction du ou des oligomères obtenus avec un polyisocyanate $OCN-D^1-NCO$, $D^1$ étant tel que défini à la revendication 1 ; et
- le cas échéant, dans une troisième étape, on conduit une réaction du produit de l'étape précédente avec un monoalcool $R^2OH$, $R^2$ étant tel que défini à la revendication 1 ;

puis, le cas échéant, on élimine le solvant.

**6.** Procédé selon la revendication 5, caractérisé par le fait qu'on conduit la réaction de Michael en faisant réagir le composé de formule

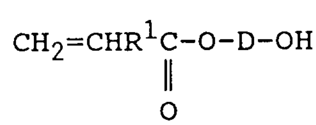

et l'acide isocyanurique à une température de 60-100 °C, en l'espace de 1-4 heures, puis on conduit une cuisson à une température de 110-156 °C, pendant 0,1-2 heures, puis on neutralise le catalyseur et on conduit l'étape de purification.

**7.** Procédé de préparation des oligomères tels que définis au point (2) de la revendication 1, dans lesquels au moins un reste A représente un groupe de formule :

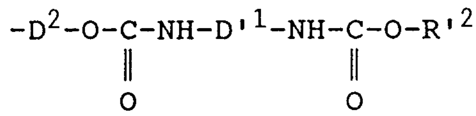 (b)

dans laquelle :

- $D^2$ est un reste alkylène en $C_1$-$C_4$ éventuellement substitué par halogène ; ou un reste -$D^3$-(-O-$D^3$-)$_n$- où $D^3$ est un reste alkylène en $C_1$-$C_4$, éventuellement substitué par halogène, et n est un nombre entier de 1 à 12 ;
- $D'^1$ est analogue à $D^1$ défini à la revendication 1 ;
- $R'^2$ est analogue à $R^2$ défini à la revendication 1 ;

le ou les autres restes A représentant

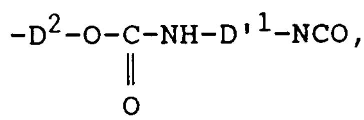

$D^2$ et $D'^1$ étant tels que définis ci-dessus,
caractérisé par le fait que :

- dans une première étape, on fait réagir un composé de formule :

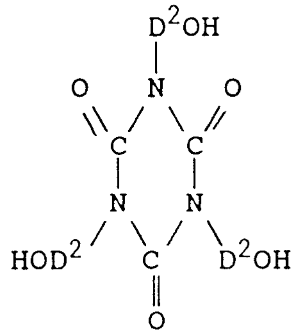

avec un polyisocyanate OCN-$D'^1$-NCO ; et

- dans une deuxième étape, on fait réagir le composé obtenu dans lequel tous les $D^2$OH ont été remplacés par

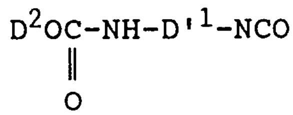

avec un composé de formule $R'^2$OH, dans un rapport molaire de 1 à 3 de $R'^2$OH au composé précédent.

**8.** Procédé selon l'une des revendications 5 à 7, caractérisé par le fait que l'on mélange les oligomères obtenus avec un acrylate multifonctionnel dans un rapport oligomère/acrylate multifonctionnel de 20 à 80% en poids.

**9.** (Co)polymère comprenant au moins un motif dérivé d'au moins un oligomère à motif isocyanurate tel que défini à l'une des revendications 1 à 3.

**Revendications pour l'Etat contractant suivant : ES**

**1.** Procédé de fabrication d'oligomères renfermant des motifs isocyanurates, représentés par la formule suivante:

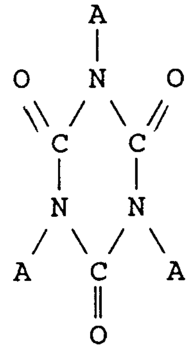

dans laquelle :

au moins un reste A représente un reste

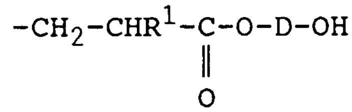 (a)

(où $R^1$ = H ou $CH_3$ et D = reste alkylène en $C_1$-$C_4$), les autres restes A représentant H ou alkyle en $C_1$-$C_4$, au moins une partie des restes A de formule (a) pouvant être transformée en restes

$A^1$ = 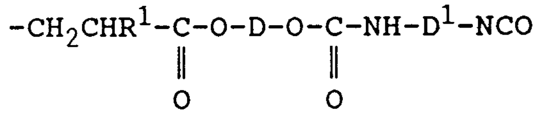

(où $D^1$ est un radical organique divalent exempt de groupes réactifs avec les groupes isocyanate et obtenu par élimination de 2 groupes NCO d'un polyisocyanate saturé ou insaturé, aromatique, aliphatique ou cycloaliphatique, contenant au moins 2 groupes NCO et ne contenant pas de groupes gênant la réaction d'un NCO avec un OH), au moins une partie des restes $A^1$ pouvant être transformée en groupes

$A^2$ = 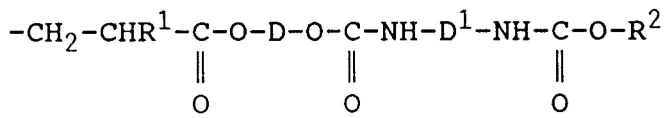

(où $R^2$ est un radical monovalent obtenu par élimination du groupe hydroxyle d'un monoalcool contenant un groupe vinylidène exempt d'autres groupes qui réagissent avec les groupes isocyanate et choisi parmi les produits de réaction d'un acide monocarboxylique contenant un groupe vinylidène avec un dialcool, $R^2$ étant alors représenté par

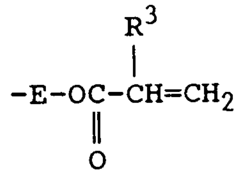

où :

- $R^3$ représente H ou $CH_3$ ; et

- E représente un motif

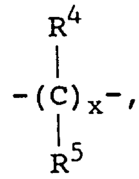

x étant un nombre entier de 1 à 12, et $R^4$ et $R^5$, identiques ou différents, étant choisis parmi l'atome d'hydrogène et des radicaux alkyle ayant 1 à 4 atomes de carbone,
et les hydroxyalkylacrylamides et hydroxyalkylméthacrylamides),
et des mélanges de ces différents composés :

caractérisé par le fait que :

- dans une première étape, on conduit une réaction de Michael entre un composé de formule

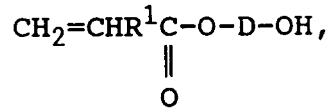

$R^1$ et D étant tels que définis ci-dessus, et l'acide isocyanurique, en catalyse basique, et dans un milieu solvant, pour obtenir un mélange d'oligomères que l'on sépare, le cas échéant, en oligomères individuels ;
- le cas échéant, dans une seconde étape, on conduit une uréthanisation par réaction du ou des oligomères obtenus avec un polyisocyanate OCN-$D^1$-NCO, $D^1$ étant tel que défini ci-dessus ; et
- le cas échéant, dans une troisième étape, on conduit une réaction du produit de l'étape précédente avec un monoalcool $R^2OH$, $R^2$ étant tel que défini ci-dessus ;

puis, le cas échéant, on élimine le solvant.

**2.** Procédé selon la revendication 1, caractérisé par le fait qu'on conduit la réaction de Michael en faisant réagir le composé de formule

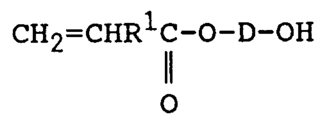

et l'acide isocyanurique à unetempérature de 60-100 °C, en l'espace de 1-4 heures, puis on conduit une cuisson à une température de 110-156 °C, pendant 0,1-2 heures, puis on neutralise le catalyseur et on conduit l'étape de purification.

**3.** Procédé de préparation d'oligomères renfermant des motifs isocyanurates, représentés par la formule suivante:

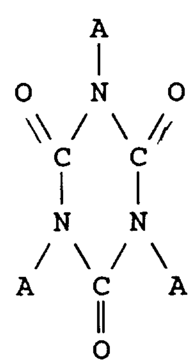

dans laquelle :

au moins un reste A représente un groupe de formule:

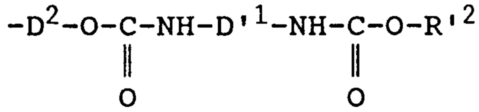 (b)

dans laquelle :

- $D^2$ est un reste alkylène en $C_1$-$C_4$ éventuellement substitué par halogène ; ou un reste -$D^3$-(-O-$D^3$-)$_n$- où $D^3$ est un reste alkylène en $C_1$-$C_4$, éventuellement substitué par halogène, et n est un nombre entier de 1 à 12 ;
- $D'^1$ est analogue à $D^1$ tel que défini à la revendication 1 ;
- $R'^2$ est analogue à $R^2$ tel que défini à la revendication 1 ;

le ou les autres restes A représentant

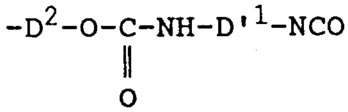,

$D^2$ et $D'^1$ étant tels que définis à la revendication 1,

et des mélanges de ces composés,

caractérisé par le fait que :

- dans une première étape, on fait réagir un composé de formule :

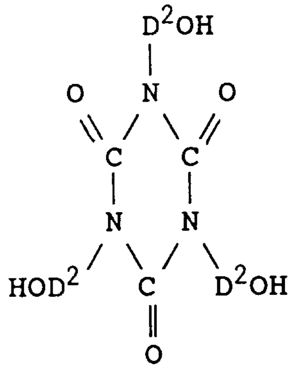

avec un polyisocyanate OCN-$D'^1$-NCO ; et

- dans une deuxième étape, on fait réagir le composé obtenu dans lequel tous les $D^2$OH ont été remplacés par

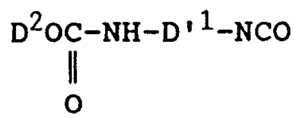

avec un composé de formule $R'^2$OH, dans un rapport molaire de 1 à 3 de $R'^2$OH au composé précédent.

**4.** Procédé selon l'une des revendications 1 à 3, caractérisé par le fait que $R^2$ ou $R'^2$ sont issus d'au moins l'un parmi le (méth)acrylate d'hydroxypropyle, le (méth)acrylate d'hydroxyéthyle, et leurs mélanges.

5. Procédé selon l'une des revendications 1 à 4, caractérisé par le fait qu'il conduit à des oligomères se présentant à l'état dilué dans un acrylate multifonctionnel, dans un rapport oligomère/acrylate multifonctionnel de 20 à 80% en poids, les oligomères obtenus étant mélangés avec un acrylate multifonctionnel dans le rapport oligomère/acrylate multifonctionnel précité.

6. Procédé de fabrication d'un (co)polymère renfermant au moins un motif dérivé d'un oligomère à motif isocyanurate, caractérisé par le fait qu'on conduit la polymérisation d'un oligomère tel qu'obtenu par le procédé selon l'une des revendications 1 à 5, ou la copolymérisation d'un tel oligomère avec un monomère copolymérisable, tel que notamment :
   - un acrylate multifonctionnel tel que défini ci-dessus,
   - un acrylate ou méthacrylate d'alkyle dont le groupe alkyle, linéaire ou ramifié, possède de 1 à 20 atomes de carbone, et
   - un acrylate ou méthacrylate d'aryle tel que le méthacrylate de benzyle,
   - un hydrocarbure vinylaromatique tel que le styrène, le vinyltoluène, l'alphaméthylstyrène, le méthyl-4 styrène, le méthyl-3 styrène, le méthoxy-4 styrène et le vinyl-1-naphtalène.

**Claims**
**Claims for the following Contracting States : AT, BE, CH, DE, DK, FR, GB, GR, IT, LI, LU, NL, SE**

1. Oligomers containing isocyanurate units, denoted by the following formula:

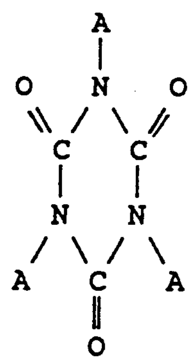

in which:
(1) at least one residue A denotes a residue

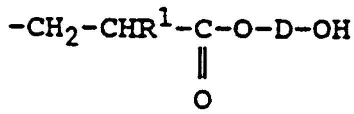 (a)

(where $R^1$ = H or $CH_3$ and D = $C_1$-$C_4$ alkylene residue), the other residues A denoting H or $C_1$-$C_4$ alkyl, it being possible for at least a part of the residues A of formula (a) to be converted into residues

$A^1$ = 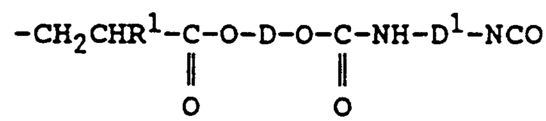

(where $D^1$ is a divalent organic radical free from groups which react with isocyanate groups and obtained by elimination of 2 NCO groups from a saturated or unsaturated, aromatic, aliphatic or cycloaliphatic polyisocyanate containing at least 2 NCO groups and not containing any group that interferes with the reaction of an NCO with an OH), it being possible for at least a part of the residues $A^1$ to be converted into groups

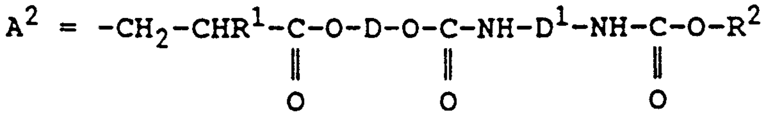

(where $R^2$ is a monovalent radical obtained by elimination of the hydroxyl group from a monoalcohol containing a vinylidene group free from other groups which react with isocyanate groups and chosen from the reaction products of a monocarboxylic acid containing a vinylidene group with a dialcohol, $R^2$ then being denoted by

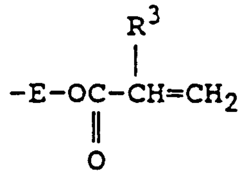

where:

- $R^3$ denotes H or $CH_3$; and
- E denotes a unit

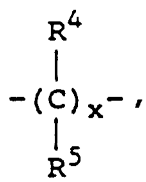

x being an integer from 1 to 12, and $R^4$ and $R^5$, which are identical or different, being chosen from the hydrogen atom and alkyl radicals containing 1 to 4 carbon atoms,

and hydroxyalkylacrylamides and hydroxyalkylmethacrylamides),

and mixtures of these different compounds;

(2) at least one residue A denotes a group of formula:

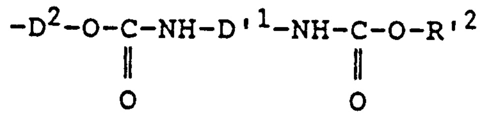 (b)

in which:

- $D^2$ is a $C_1$-$C_4$ alkylene residue optionally substituted by halogen; or a residue $-D^3-(-O-D^3-)_n-$ where$D^3$ is a $C_1$-$C_4$ alkylene residue optionally substituted by halogen, and n is an integer from 1 to 12;
- $D'^1$ is analogous to $D^1$ defined above;
- $R'^2$ is analogous to $R^2$ defined above,

the other residue(s) A denoting

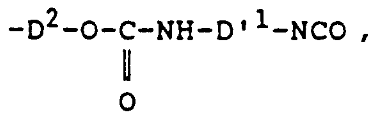

$D^2$ and $D'^1$ being as defined above, $D^2$ being incapable of denoting a $C_1$-$C_4$ alkylene residue in the case where the oligomer carries the three groups (b),

and the mixtures of these compounds.

2. Oligomers according to Claim 1, characterized in that $D^1$ is a radical obtained by elimination of 2 NCO groups from an aromatic polyisocyanate.

3. Oligomers according to either of Claims 1 and 2 characterized in that $R^2$ or $R'^2$ originates from at least one out of hydroxypropyl (meth)acrylate, hydroxyethyl (meth)acrylate and their mixtures.

4. Oligomers according to one of Claims 1 to 3, characterized in that they are in the dilute state in a multifunctional acrylate, in an oligomer/multifunctional acrylate ratio of 20 to 80% by weight.

5. Process for the preparation of the oligomers as defined in point (1) of Claim 1, characterized in that:
   - in a first stage a Michael reaction is carried out between a compound of formula

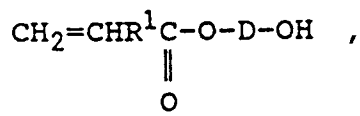

   $R^1$ and D being as defined in Claim 1, and isocyanuric acid, with basic catalysis and in a solvent medium, to obtain a mixture of oligomers which are separated, if appropriate, into individual oligomers;
   - if appropriate, in a second stage, a urethanization is carried out by reacting the oligomer(s) obtained with a polyisocyanate OCN-$D^1$-NCO, $D^1$ being as defined in Claim 1; and
   - if appropriate, in a third stage, a reaction of the product from the preceding stage is carried out with a monoalcohol $R^2OH$, $R^2$ being as defined in Claim 1;

   then, if appropriate, the solvent is removed.

6. Process according to Claim 5, characterized in that the Michael reaction is carried out by reacting the compound of formula

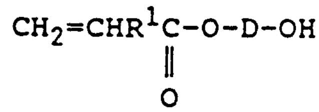

   and isocyanuric acid at a temperature of 60-100 °C over a period of 1-4 hours, heating at a temperature of 110-156 °C is then carried out for 0.1-2 hours, the catalyst is then neutralized and the purification stage is carried out.

7. Process for the preparation of the oligomers as defined in point (2) of Claim 1, in which at least one residue A denotes a group of formula:

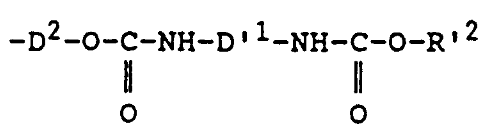 (b)

   in which:
   - $D^2$ is a $C_1$-$C_4$ alkylene residue optionally halogen-substituted; or a residue -$D^3$-(-O-$D^3$-)$_n$-where $D^3$ is a $C_1$-$C_4$ alkylene residue optionally halogen-substituted, and n is an integer from 1 to 12;
   - $D'^1$ is analogous to $D^1$ defined in Claim 1;
   - $R'^2$ is analogous to $R^2$ defined in Claim 1,

   the other residue(s) A denoting

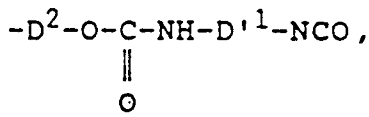

$D^2$ and $D'^1$ being as defined above, characterized in that:

- in a first stage, a compound of formula:

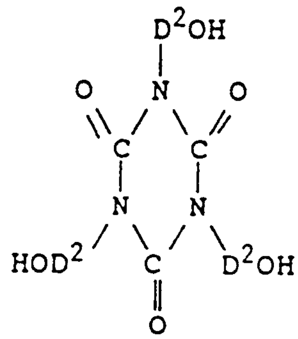

  is reacted with a polyisocyanate OCN-$D'^1$-NCO; and
- in a second stage, the compound obtained, in which all the $D^2OH$ have been replaced with

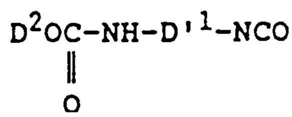

,

  is reacted with a compound of formula $R'^2OH$, in a molar ratio of 1 to 3 of $R'^2OH$ to the preceding compound.

**8.** Process according to one of Claims 5 to 7, characterized in that the oligomers obtained are mixed with a multifunctional acrylate in an oligomer/multifunctional acrylate ratio of 20 to 80% by weight.

**9.** (Co)polymer including at least one unit derived from at least one oligomer containing an isocyanurate unit as defined in one of Claims 1 to 3.

**Claims for the following Contracting State : ES**

**1.** Process for the manufacture of oligomers containing isocyanurate units, denoted by the following formula:

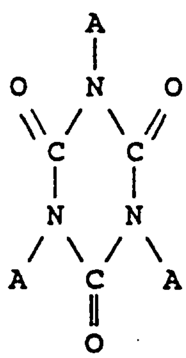

in which:

at least one residue A denotes a residue

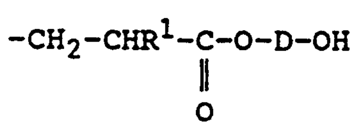

(a)

(where $R^1$ = H or $CH_3$ and D = $C_1$-$C_4$ alkylene residue), the other residues A denoting H or $C_1$-$C_4$ alkyl, it being possible for at least a part of the residues A of formula (a) to be converted into residues

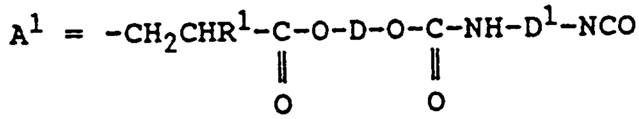

(where $D^1$ is a divalent organic radical free from groups which react with isocyanate groups and obtained by elimination of 2 NCO groups from a saturated or unsaturated aromatic, aliphatic or cycloaliphatic polyisocyanate containing at least 2 NCO groups and not containing any group that interferes with the reaction of an NCO with an OH), it being possible for at least a part of the residues $A^1$ to be converted into groups

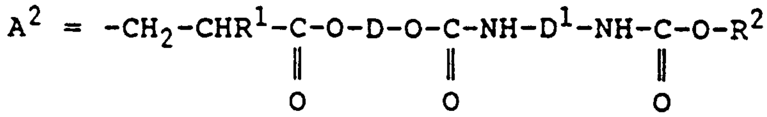

(where $R^2$ is a monovalent radical obtained by elimination of the hydroxyl group from a monoalcohol containing a vinylidene group free from other groups which react with isocyanate groups and chosen from the reaction products of a monocarboxylic acid containing a vinylidene group with a dialcohol, $R^2$ then being denoted by

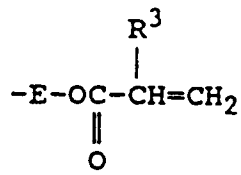

where:

- $R^3$ denotes H or $CH_3$; and
- E denotes a unit

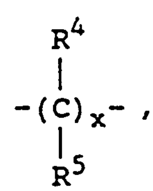

x being an integer from 1 to 12, and $R^4$ and $R^5$, which are identical or differ ent, being chosen from the hydrogen atom and alkyl radicals containing 1 to 4 carbon atoms

and hydroxyalkylacrylamides and hydroxyalkylmethacrylamides),
and mixtures of these different compounds;
characterized in that:

- in a first stage a Michael reaction is carried out between a compound of formula

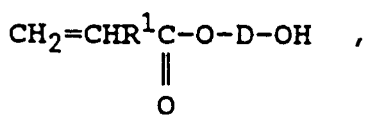

$R^1$ and D being as defined above, and isocyanuric acid, with basic catalysis and in a solvent medium, to obtain a mixture of oligomers which are separated, if appropriate, into individual

oligomers;

- if appropriate, in a second stage, a urethanization is carried out by reacting the oligomer(s) obtained with a polyisocyanate $OCN-D^1-NCO$, $D^1$ being as defined above; and
- if appropriate, in a third stage, a reaction of the product from the preceding stage is carried out with a monoalcohol $R^2OH$, $R^2$ being as defined above;

then, if appropriate, the solvent is removed.

**2.** Process according to Claim 1, characterized in that the Michael reaction is carried out by reacting the compound of formula

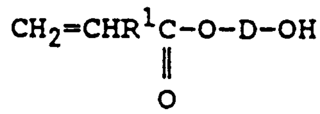

and isocyanuric acid at a temperature of 60-100 °C over a period of 1-4 hours, heating at a temperature of 110-156 °C is then carried out for 0.1-2 hours, the catalyst is then neutralized and the purification stage is carried out.

**3.** Process for the preparation of oligomers containing isocyanurate units denoted by the following formula:

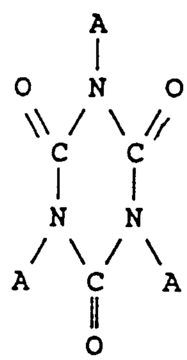

in which:

at least one residue A denotes a group of formula:

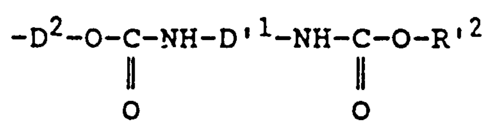 (b)

in which:

- $D^2$ is a $C_1$-$C_4$ alkylene residue optionally halogen-substituted; or a residue $-D^3-(-O-D^3-)_n-$where $D^3$ is a $C_1$-$C_4$ alkylene residue optionally halogen-substituted, and n is an integer from 1 to 12;
- $D'^1$ is analogous to $D^1$ as defined in Claim 1;
- $R'^2$ is analogous to $R^2$ as defined in Claim 1;

the other residue(s) A denoting

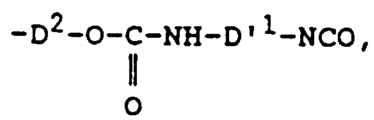

$D^2$ and $D'^1$ being as defined in Claim 1,

and mixtures of these compounds,
characterized in that:

- in a first stage, a compound of formula:

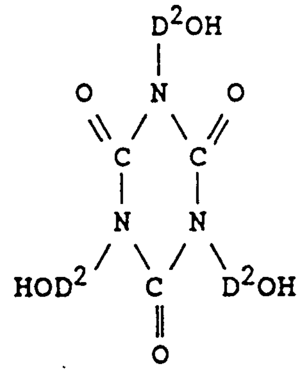

is reacted with a polyisocyanate OCN-$D'^1$-NCO; and

- in a second stage, the compound obtained, in which all the $D^2OH$ have been replaced with

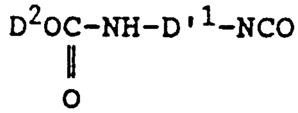

,

is reacted with a compound of formula $R'^2OH$, in a molar ratio of 1 to 3 of $R'^2OH$ to the preceding compound.

**4.** Process according to one of Claims 1 to 3, characterized in that $R^2$ or $R'^2$ originates from at least one out of hydroxypropyl (meth)acrylate, hydroxyethyl (meth)acrylate and their mixtures.

**5.** Process according to one of Claims 1 to 4, characterized in that it results in oligomers which are in the dilute state in a multifunctional acrylate, in an oligomer/multifunctional acrylate ratio of 20 to 80% by weight, the oligomers obtained being mixed with a multifunctional acrylate in the abovementioned oligomer/multifunctional acrylate ratio.

**6.** Process for the manufacture of a (co)polymer including at least one unit derived from an oligomer containing an isocyanurate unit, characterized in that the polymerization is carried out of an oligomer such as is obtained by the process according to one of Claims 1 to 5, or the copolymerization is carried out of such an oligomer with a monomer which can be copolymerized, such as especially:

- a multifunctional acrylate as defined above,
- an alkyl acrylate or methacrylate in which the linear or branched alkyl group contains from 1 to 20 carbon atoms, and
- an aryl acrylate or methacrylate such as benzyl methacrylate,
- a vinyl aromatic hydrocarbon such as styrene, vinyltoluene, alpha-methylstyrene, 4-methylstyrene, 3-methylstyrene, 4-methoxystyrene and 1-vinylnaphthalene.

## Patentansprüche

**Patentansprüche für folgende Vertragsstaaten : AT, BE, CH, DE, DK, FR, GB, GR, IT, LI, LU, NL, SE**

**1.** Oligomere mit Isocyanurat-Einheiten der folgenden Formel,

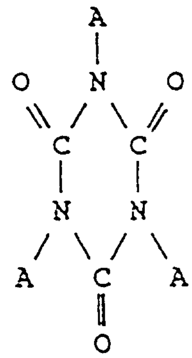

,

in der bedeuten:

(1) mindestens ein Rest A eine Gruppe der Formel

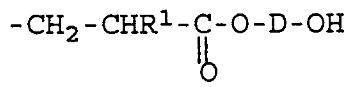

(a)

(worin $R^1$ H oder $CH_3$ und
D eine $C_1$-$C_4$-Alkylengruppe bedeuten),
wobei die übrigen Reste A H oder $C_1$-$C_4$-Alkyl darstellen und wobei mindestens ein Teil der Reste A der Formel (a) in Gruppen $A^1$ der Formel

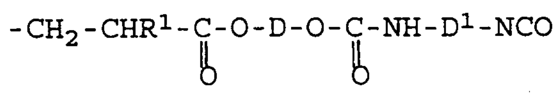

übergeführt sein kann (worin $D^1$ eine zweiwertige organische Gruppe darstellt, die keine gegenüber Isocyanatgruppen reaktiven Gruppen aufweist, und die durch Entfernung von 2 NCO-Gruppen von einem gesättigten oder ungesättigten aromatischen, aliphatischen oder cycloaliphatischen Polyisocyanat, das mindestens 2 NCO-Gruppen enthält und keine die Reaktion von NCO mit OH störenden Gruppen aufweist, erhalten ist),
wobei mindestens ein Teil der Reste $A^1$ in Gruppen $A^2$ der Formel

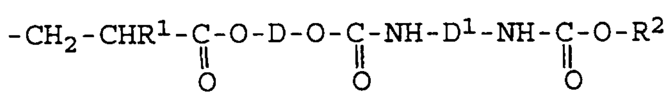

übergeführt sein kann (worin $R^2$ eine einwertige Gruppe darstellt, die durch Entfernung der Hydroxylgruppe von einem einwertigen, eine Vinylidengruppe ententhaltenden Alkohol erhalten ist, der keine weiteren, mit Isocyanatgruppen reagierende Gruppen aufweist und unter den Reaktionsprodukten der Reaktion einer eine Vinylidengruppe enthaltenden Monocarbonsäure mit einem zweiwertigen Alkohol, wobei $R^2$ dann der Formel

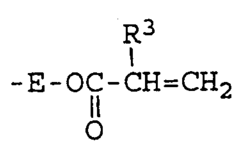

entspricht,
worin bedeuten:

- $R^3$ H oder $CH_3$

und

- E eine Gruppe

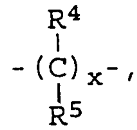

wobei x eine ganze Zahl von 1 bis 12 bedeutet und $R^4$ und $R^5$, die gleich oder verschieden sein können, unter Wasserstoff und $C_1$-$C_4$-Alkylgruppen ausgewählt sind,

und den Hydroxyalkylacrylamiden und Hydroxyalkylmethacrylamiden ausgewählt ist),
sowie die Gemische dieser verschiedenen Verbindungen;
(2) mindestens ein Rest A eine Gruppe der Formel

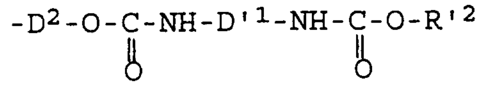 (b),

in der bedeuten:

- $D^2$ eine gegebenenfalls halogensubstituierte $C_1$-$C_4$-Alkylengruppe oder eine Gruppe $-D^3-(-O-D^3-)_n-$ in der $D^3$ eine gegebenenfalls halogensubstituierte $C_1$-$C_4$-Alkylengruppe und n eine ganze Zahl von 1 bis 12 bedeuten;
- $D'^1$ dasselbe wie oben für $D^1$ definiert;
- $R'^2$ dasselbe wie oben für $R^2$ definiert,

wobei der andere Rest A bzw. die anderen Reste A Gruppen der Formel

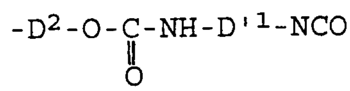

darstellen, worin $D^2$ und $D'^1$ wie oben definiert sind und $D^2$ keine $C_1$-$C_4$-Alkylengruppe darstellen kann, wenn das Oligomer drei Gruppen der Formel (b) aufweist,
sowie die Gemische dieser Verbindungen.

**2.** Oligomere nach Anspruch 1, dadurch gekennzeichnet, daß $D^1$ eine durch Entfernung von 2 NCO-Gruppen von einem aromatischen Polyisocyanat erhaltene Gruppe ist.

**3.** Oligomere nach Anspruch 1 oder 2, dadurch gekennzeichnet, daß $R^2$ oder $R'^2$ von mindestens einem Vertreter der aus Hydroxypropylacrylat, Hydroxypropylmethacrylat, Hydroxyethylacrylat, Hydroxyethylmethacrylat sowie deren Gemischen bestehenden Gruppe stammen.

**4.** Oligomere nach einem der Ansprüche 1 bis 3, dadurch gekennzeichnet, daß sie in in einem multifunktionellen Acrylat verdünntem Zustand vorliegen, wobei das Verhältnis oligomer/multifunktionelles Acrylat 20 bis 80 Gew.-% beträgt.

**5.** Verfahren zur Herstellung der Oligomeren nach Anspruch 1 (1), dadurch gekennzeichnet, daß:

- in einer ersten Stufe eine Michael-Addition zwischen einer Verbindung der Formel

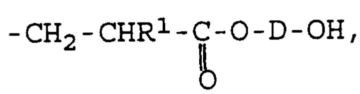

in der $R^1$ und D wie in Anspruch 1 definiert sind, und Isocyanursäure unter basischer Katalyse in einem Lösungsmittelmedium durchgeführt wird, wobei ein Oligomerengemisch erhalten wird, das erforderlichenfalls in die individuellen Oligomeren aufgetrennt wird;

- erforderlichenfalls in einer zweiten Stufe durch Umsetzung des oder der erhaltenen Oligomeren mit einem Polyisocyanat der Formel OCN-$D^1$-NCO, worin $D^1$ wie in Anspruch 1 definiert ist, eine Urethanisierung durchgeführt wird,

und

- erforderlichenfalls in einer dritten Stufe das Produkt der vorhergehenden Stufe mit einem einwertigen Alkohol der Formel $R^2OH$, in der $R^2$ wie in Anspruch 1 definiert ist, umgesetzt wird,
- sowie gegebenenfalls anschließend das Lösungsmittel abgetrennt wird.

**6.** Verfahren nach Anspruch 5, dadurch gekennzeichnet, daß die Michael-Addition durch Umsetzung der Verbindung der Formel

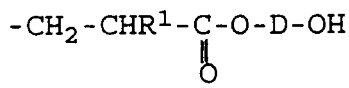

mit Isocyanursäure bei einer Temperatur von 60 bis 100 °C während 1 bis 4 h durchgeführt wird, das Produkt anschließend 0,1 bis 2 h auf 110 bis 156 °C gehalten wird und anschließend der Katalysator neutralisiert und das Produkt gereinigt wird.

**7.** Verfahren zur Herstellung der Oligomeren nach Anspruch 1 (2), bei denen mindestens ein Rest A einer Gruppe der Formel

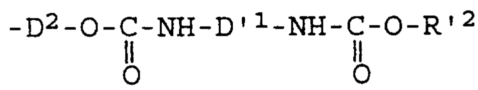 (b)

darstellt, in der bedeuten:

- $D^2$ eine gegebenenfalls halogensubstituierte $C_1$-$C_4$-Alkylengruppe oder eine Gruppe $-D^3-(-O-D^3-)_n-$, in der $D^3$ eine gegebenenfalls halogensubstituierte $C_1$-$C_4$-Alkylengruppe und n eine ganze Zahl von 1 bis 12 bedeuten;
- $D'^1$ dasselbe wie oben für $D^1$ definiert;
- $R'^2$ dasselbe wie oben für $R^2$ definiert,

wobei der andere Rest A bzw. die anderen Reste A Gruppen der Formel

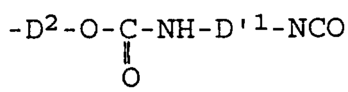

darstellen, worin $D^2$ und $D'^1$ wie oben definiert sind,
dadurch gekennzeichnet, daß:

- in einer ersten Stufe eine Verbindung der Formel

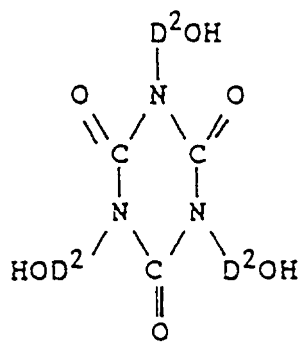

mit einem Polyisocyanat der Formel OCN-D'[1]-NCO umgesetzt wird und in einer zweiten Stufe die erhaltene Verbindung, in deren Formel sämtliche $D^2OH$ durch

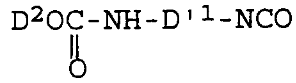

ersetzt sind, mit einer Verbindung der Formel $R'^2OH$ in einem Molverhältnis von 1 bis 3 $R'^2OH$, bezogen auf die vorhergehende Verbindung, umgesetzt wird.

**8.** Verfahren nach einem der Ansprüche 5 bis 7, dadurch gekennzeichnet, daß die erhaltenen Isomeren mit einem multifunktionellen Acrylat in einem Verhältnis von Oligomer/multifunktionelles Acrylat von 20 bis 80 Gew.-% gemischt werden.

**9.** Polymere bzw. Copolymere, die mindestens eine Einheit aufweisen, die von mindestens einem Oligomer mit Isocyanurateinheit, wie in einem der Ansprüche 1 bis 3 definiert, abgeleitet ist.

**Patentansprüche für folgenden Vertragsstaat : ES**

**1.** Verfahren zur Herstellung von Oligomeren mit Isocyanurat-Einheiten der folgenden Formel:

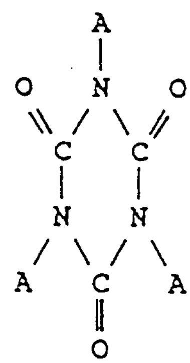

,

in der bedeuten:

mindestens ein Rest A eine Gruppe der Formel

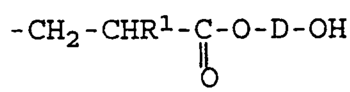 (a)

(worin $R^1$ H oder $CH_3$ und
D eine $C_1$-$C_4$-Alkylengruppe bedeuten),
wobei die übrigen Reste A H oder $C_1$-$C_4$-Alkyl darstellen und wobei mindestens ein Teil der Reste A der Formel (a) in Gruppen $A^1$ der Formel

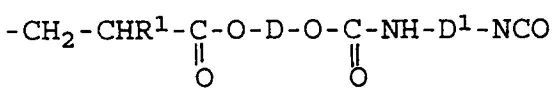

übergeführt sein kann (worin $D^1$ eine zweiwertige organische Gruppe darstellt, die keine gegenüber Isocyanatgruppen reaktiven Gruppen aufweist, und die durch Entfernung von 2 NCO-Gruppen von einem gesättigten oder ungesättigten aromatischen, aliphatischen oder cycloaliphatischen Polyisocyanat, das mindestens 2 NCO-Gruppen enthält und keine die Reaktion von NCO mit OH störenden Gruppen aufweist, erhalten ist),
wobei mindestens ein Teil der Reste $A^1$ in Gruppen $A^2$ der Formel

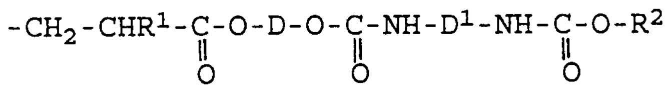

übergeführt sein kann (worin $R^2$ eine einwertige Gruppe darstellt, die durch Entfernung der Hydroxylgruppe von einem einwertigen, eine Vinylidengruppe ententhaltenden Alkohol erhalten ist, der keine weiteren, mit Isocyanatgruppen reagierende Gruppen aufweist und unter den Reaktionsprodukten der Reaktion einer eine Vinylidengruppe enthaltende Monocarbonsäure mit einem zweiwertigen Alkohol, wobei $R^2$ dann der Formel

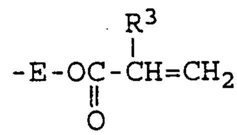

entspricht,
worin bedeuten:
- $R^3$ H oder $CH_3$

und
- E eine Gruppe

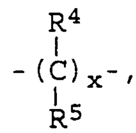

wobei x eine ganze Zahl von 1 bis 12 bedeutet und $R^4$ und $R^5$, die gleich oder verschieden sein können, unter Wasserstoff und $C_1$-$C_4$-Alkylgruppen ausgewählt sind,

und den Hydroxyalkylacrylamiden und Hydroxyalkylmethacrylamiden ausgewählt ist),
sowie die Gemische dieser verschiedenen Verbindungen,
dadurch gekennzeichnet, daß:
- in einer ersten Stufe eine Michael-Addition zwischen einer Verbindung der Formel

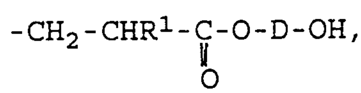

in der $R^1$ und D wie oben definiert sind, und Isocyanursäure unter basischer Katalyse in einem Lösungsmittelmedium durchgeführt wird, wobei ein Oligomerengemisch erhalten wird, das erforderlichenfalls in die individuellen Oligomeren aufgetrennt wird;
- erforderlichenfalls in einer zweiten Stufe durch Umsetzung des oder der erhaltenen Oligomeren mit einem Polyisocyanat der Formel OCN-$D^1$-NCO, worin $D^1$ wie oben definiert ist, eine Urethanisierung durchgeführt wird,

und
- erforderlichenfalls in einer dritten Stufe das Produkt der vorhergehenden Stufe mit einem einwertigen Alkohol der Formel $R^2OH$, in der $R^2$ wie oben definiert ist, umgesetzt wird,
- sowie gegebenenfalls anschließend das Lösungsmittel abgetrennt wird.

**2.** Ein Verfahren nach Anspruch 1, dadurch gekennzeichnet, daß die Michael-Addition durch Umsetzung der Verbindung der Formel

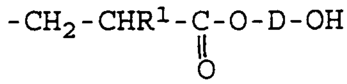

mit Isocyanursäure bei einer Temperatur von 60 bis 100 °C während 1 bis 4 h durchgeführt wird, das Produkt anschließend 0,1 bis 2 h auf 110 bis 156 °C gehalten wird und anschließend der Katalysator neutralisiert und das Produkt gereinigt wird.

**3.** Verfahren zur Herstellung von Oligomeren mit Isocyanurat-Einheiten der folgenden Formel,

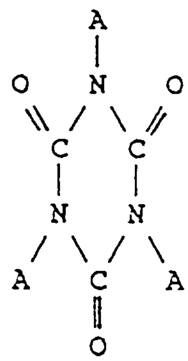

,

in der bedeuten:
mindestens ein Rest A eine Gruppe der Formel

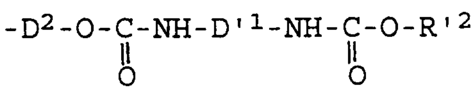

(b),

in der bedeuten:

- $D^2$ eine gegebenenfalls halogensubstituierte $C_1$-$C_4$-Alkylengruppe oder eine Gruppe $-D^3-(-O-D^3-)_n-$ in der $D^3$ eine gegebenenfalls halogensubstituierte $C_1$-$C_4$-Alkylengruppe und n eine ganze Zahl von 1 bis 12 bedeuten;
- $D'^1$ dasselbe wie in Anspruch 1 für $D^1$ definiert;
- $R'^2$ dasselbe wie in Anspruch 1 für $R^2$ definiert,

wobei der andere Rest A bzw. die anderen Reste A Gruppen der Formel

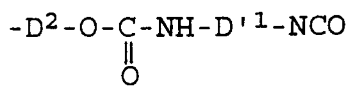

darstellen, worin $D^2$ und $D'^1$ wie in Anspruch 1 definiert sind,
und der Gemische dieser Verbindungen,
dadurch gekennzeichnet, daß:
in einer ersten Stufe eine Verbindung der Formel

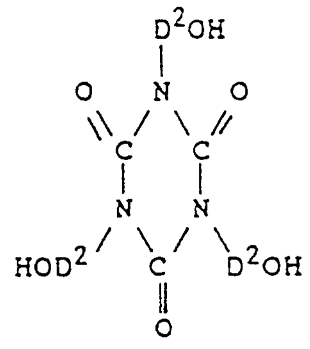

mit einem Polyisocyanat der Formel OCN-D'1-NCO umgesetzt wird und in einer zweiten Stufe die erhaltene Verbindung, in deren Formel sämtliche $D^2OH$ durch

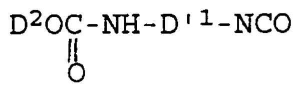

ersetzt sind, mit einer Verbindung der Formel $R'^2OH$ in einem Molverhältnis von 1 bis 3 $R'^2OH$, bezogen auf die vorhergehende Verbindung, umgesetzt wird.

**4.** Verfahren nach einem der Ansprüche 1 bis 3, dadurch gekennzeichnet, daß $R^2$ oder $R'^2$ von mindestens einem Vertreter der aus Hydroxypropylacrylat, Hydroxypropylmethacrylat, Hydroxyethylacrylat, Hydroxyethylmethacrylat sowie deren Gemischen bestehenden Gruppe stammen.

**5.** Verfahren nach einem der Ansprüche 1 bis 4, dadurch gekennzeichnet, daß es zu Oligomeren führt, die in in einem multifuntionellen Acrylat verdünntem Zustand vorliegen, wobei das Verhältnis Oligomer/multifunktionelles Acrylat 20 bis 80 Gew.-% beträgt, und die ererhaltenen Oligomeren mit einem multifunktionellen Acrylat im oben angegebenen Verhältnis Oligomer/multifunktionelles Acrylat gemischt werden.

**6.** Verfahren zur Herstellung von Polymeren bzw. Copolymeren, die mindestens eine Einheit aufweisen, die von einem Oligomer mit einer Isocyanurat-Einheit abgeleitet ist, gekennzeichnet durch Polymerisation eines Oligomers wie nach einem der Ansprüch 1 bis 5 erhalten oder Copolymerisation eines derartigen Oligomers mit einem copolymerisierbaren Monomer, wie insbesondere:

- einem multifunktionellen Acrylat wie oben definiert,
- einem Alkylacrylat oder Alkylmethacrylat, dessen Alkylgruppe, die geradkettig oder verzweigt sein kann, 1 bis 20 Kohlenstoffatome aufweist, und
- einem Arylacrylat oder Arylmethacrylat, wie Benzylmethacrylat,
- einem vinylaromatischen Kohlenwasserstoff, wie Styrol, Vinyltoluol, $\alpha$-Methylstyrol, 4-Methylstyrol, 3-Methylstyrol, 4-Methoxystyrol und 1-Vinylnaphthalin.